# EUROPEAN PATENT SPECIFICATION

(11) **EP 3 218 095 B1**
(45) Date of publication and mention of the grant of the patent: **31.10.2018**
(21) Application number: 15794162.6
(22) Date of filing: 12.11.2015
(51) Int. Cl.: B01F 11/00, B01F 15/00

(54) **ORBITAL SHAKER**
ORBITALSCHÜTTLER
SECOUEUR ORBITAL

(30) Priority: 13.11.2014 EP 14193009
(43) Date of publication of application: 20.09.2017
(73) Proprietor: Enzyscreen B.V., 2105 MB Heemstede (NL)
(72) Inventor: DUETZ, Wouter Adriaan, NL-2105 MB Heemstede (NL)
(74) Representative: DeltaPatents B.V.
(86) International application number: PCT/EP2015/076386
(87) International publication number: WO 2016/075214

(56) References cited:
- JP-A- H0 747 255
- US-A- 5 052 812
- US-A- 5 372 425

## Description

### Field of the Invention

The present invention relates to an orbital shaker. More in particular, the present invention relates to an orbital shaking device for biotechnological and/or biomedical applications, for example stimulating, sustaining or monitoring microbial and eukaryotic cell growth.

### Background of the Invention

In several fields of technology, fluids and other substances have to be shaken. In biotechnology and biomedical applications, for example, orbital shaking is often used for microbial and cell cultures growing in e.g. Erlenmeyer shake flasks or microtitre plates (also known as microwell plates or microplates), in order to achieve an increased gas-liquid exchange rate, and so an increased oxygen input and CO₂ outgassing. In biotechnology applications, shaking amplitudes typically range between 3 and 50 mm, and shaking frequencies between 150 and 1200 rpm (that is, between 2.5 and 20 Hz).

Commercially available orbital shakers for biotechnological applications in laboratory settings generally have either (i) a three-point shaking system consisting of three bearings with eccentric axes in a triangle under the shaker platform, one of the axes being driven by a motor, or (ii) a central drive, in combination with a parallelogram. In both cases, internal counterweights are positioned directly under the shaker platform, which results in (full or partial) equilibration in the horizontal plane, and an incomplete (but mostly acceptable) equilibration in the vertical plane.

European Patent EP 2 301 679 discloses an orbital shaker provided with counterweights both above and below the orbitally shaking platform. An embodiment comprises four counterweights rotating around two vertical axes. The trajectories of the two lower counterweights cover most of the underside of the platform. In another embodiment the trajectory of a single lower counterweight extends over substantially all of the underside of the platform.

A disadvantage of the shaker of EP 2 301 679, and of the commercially available orbital shakers mentioned above, that they have both moving and stationary parts underneath the platform. It is often desired to monitor microbial and eukaryotic cell cultures which are being shaken, for example to monitor their growth. It is also desired to supply cultures, for example algae, with a suitable amount of light. To this end, it is desired to put imaging devices, such as scanners and/or cameras, and/or illumination devices, such as lamps, underneath the platform which is, for this purpose, made transparent or provided with suitable openings. However, mounting imaging devices and/or illumination devices underneath the platform is typically not possible with prior art devices due to the presence of the drive system and/or the counterweights.

Another disadvantage of the shaker of EP 2 301 679 is that only one axis is driven by the motor, the other axes being indirectly driven by the platform. Practice has shown that such arrangements are subject to significant stress, and hence to significant wear, when used at the shaking frequencies which are common in biotechnical applications (typically about 150 to 1200 rpm). It is especially at the higher rotational speeds that this wear hampers the long-term reliability of the shaking device. Also the commercially available shaker devices mentioned above, having a three-point system or a central drive with a parallelogram, are known to be prone to failure, especially at higher speeds (above 800 rpm).

United States patent US 5 372 425 discloses a shaker apparatus in accordance with the preamble of claim 1, provided with drive means located centrally underneath the platform. The shaker apparatus of US 5 372 425 is therefore unsuitable for mounting imaging devices and/or illumination devices underneath the platform.

### Summary of the Invention

It is an object of the present invention to solve these and other problems of the state of the art by providing an orbital shaker device which allows room for an imaging and/or illumination apparatus (or apparatuses) underneath the moving platform while being able to operate at shaking frequencies in the biotechnological range, that is, between approximately 150 and 1200 rpm.

It is a further object of the present invention to provide an orbital shaker which allows the counterweights to be adapted to the load in order to reduce or substantially eliminate vibrations at varying numbers of containers.

Accordingly, the present invention provides an orbital shaker device for biotechnological and/or biomedical applications, comprising:
- a frame,
- a platform for receiving biotechnological and/or biomedical containers,
- eccentric couplings for allowing an orbital movement of the platform relative to the frame,
- counterweight units for balancing the orbital movement, and
- at least one motor for driving the eccentric couplings, wherein
- the device comprises two eccentric couplings arranged near respective opposite edges of the platform, wherein
- each counterweight unit is arranged approximately in the plane of the platform, and
- both eccentric couplings are driven by the at least one motor.

By providing two eccentric couplings near two edges of the platform, the couplings are spaced apart from the containers receiving part of the platform, and hence from the bottom side of the containers receiving part. As a result, the axes and/or motors driving the couplings can also be spaced apart from the containers receiving part of the platform. In addition, providing two instead of three or more eccentric couplings results in a simpler construction and a reduced wear.

By positioning the counterweight units approximately in the plane of the platform, or more specifically, in the plane of the combined center of gravity of the platform and the containers when in use, that is, approximately in the plane of the center of gravity of the loaded platform when in use, not only the horizontal balancing is optimized, but also the vertical balancing, as the counterweights are at the same height as the combination of the platform and its load. In addition, mounting the counterweights both in approximately the plane of the center of gravity of the platform when loaded with containers and near edges of the platform ensures that the counterweights are not located underneath the platform, thus keeping the bottom side of the platform free for imaging and/or illumination apparatus(es) and other purposes.

By driving both eccentric couplings instead of only one, a better synchronization of the couplings, a reduced amount of vibrations and a significantly reduced wear are achieved.

It is noted that German Patent DE 33 19 574 discloses a shaking device in which a counterweight is located just beneath the platform in order to bring the counterweight as close as possible to the center of gravity of the loaded platform. However, by mounting the counterweight just beneath the platform, a vertical imbalance will unavoidably remain. In addition, the position of the counterweight will hinder taking images of the platform from underneath.

It is further noted that International Patent Application WO 2010/037862 discloses an orbital shaking device provided with a flatbed scanner for generating two-dimensional images of the bottom sides of microtitre plates. WO 2010/037862 discloses no details of the shaking mechanism.

In the device of the present invention, the containers are preferably biotechnological containers, such as microplates, laboratory beakers and/or Erlenmeyer flasks. In the case of microplates, the containers will be relatively shallow and have a limited height. When the platform is receiving a single layer of relatively shallow containers, the combined center of gravity will be only slightly above the platform and may even substantially coincide with the plane of the platform.

The at least one motor may drive the eccentric couplings in several ways. In an embodiment, each eccentric coupling is driven by a respective vertical axis, the axes being driven by a single motor. That is, both axes are driven by a single motor, using a mechanism for transferring the rotation of the motor to the eccentric couplings via the vertical axes on which the eccentric couplings are mounted. In one such embodiment, the single motor is connected to the axes through right-angle gear boxes. This allows the motor to be arranged horizontally, having for example horizontal drive axes extending at both ends of the motor, which drive axes are coupled to the vertical axes by means of the right-angle gear boxes. Such an arrangement allows the motor to be arranged at almost any convenient distance from the platform, thus providing ample space for an imaging and/or illumination apparatus (or apparatuses). Alternatively, the single motor may be connected to the vertical axes through a belt, in combination with suitable wheels for driving the belt and the vertical axes. When using a belt, it is preferred to use a toothed belt to avoid slip and thereby guarantee the synchronous rotation of the eccentric couplings.

Instead of using a single motor for driving all eccentric couplings, each eccentric coupling may be driven by a separate motor. Such an embodiment removes the need for additional axes or a belt, but requires an additional motor. Particularly in such an embodiment, the device preferably comprising sensors for sensing the speed of the motors. Such sensors allow the rotational speed of the motors to be kept equal, thereby ensuring a synchronous rotation of the eccentric couplings. To this end, the device of the present invention may further comprise a control unit for controlling the speed of the motors, preferably in response to the sensed speed. A speed control unit may also be advantageous in embodiments having a single motor.

In order to monitor the growth (or death) of microorganism cultures or eukaryotic cell cultures, the device of the present invention may comprise an imaging unit located underneath the platform, said platform preferably being provided with openings and/or transparent parts allowing imaging the containers. The imaging unit may comprise a single camera or an array of cameras. Alternatively, or additionally, the imaging unit may comprise a scanner or other imaging means. The imaging unit may further comprise a processor for image processing and analysis. The processor uses techniques which may be known per se.

In a particularly advantageous embodiment, the control unit is arranged for reducing the speed of the at least one motor in preparation for imaging. That is, the shaking frequency is reduced when an image, or a series of images, is to be made. This allows better quality images to be made. The motor, and hence the platform, may come to a standstill when an image is made, but this is only required when using a scanner and is not required when using a camera having a relatively short exposure time. It will be understood that the motor speed is brought back to its original value when the imaging is completed.

When a single layer of relatively low containers is used, counterweight units having a fixed mass are typically suitable. However, when higher containers are used, or containers having relatively large variation in the weight of their contents, or when containers are stacked, then counterweight units exerting a fixed centrifugal force at a certain speed are often less suitable. In a particularly advantageous embodiment, therefore, the counterweight units are adjustable. That is, the mass of the counterweights can be increased or decreased, or be moved relative to the eccentric couplings so as to have a greater or lesser counterbalancing effect. In an embodiment, therefore the counterweight units are arranged for adding and/or removing counterweights. In another embodiment, the counterweight units may comprise counterweights having an asymmetrical mass distribution relative to an axis and which can be rotated about said axis, so as to increase or decrease their counterbalancing effect. The centrifugal force at a certain rotational speed may alternatively, or additionally, be increased or decreased by changing the distance between a counterweight's center of gravity and the axis around which it rotates.

The platform may be arranged for stacking containers and may for example be provided with support members to facilitate stacking and to prevent stacks of containers from falling over.

Although the device may have a plurality of eccentric couplings for rotatably coupling the platform to the frame, for example four, in a preferred embodiment the device has only two eccentric couplings. This limited number of joints ensures a proper functioning of the device with a minimum number of parts.

The present invention also provides a use of an orbital shaker device as defined above when provided with adjustable counterweight units, the use comprising the step of adjusting the counterweight units. The use may further comprise the step of adding or removing counterweights, in particular when placing a load on the platform, or when increasing or decreasing the load of the platform.

### Brief description of the drawings

The present invention will further be explained with reference to exemplary embodiments illustrated in the drawings, in which:
Fig. 1 schematically shows a side view of an embodiment of a device according to the present invention.
Fig. 2 schematically shows an end view of the embodiment illustrated in Fig. 2.
Fig. 3 schematically shows a perspective view of the embodiment illustrated in Fig. 2.
Fig. 4 schematically shows a top view of the embodiment illustrated in Fig. 2.
Fig. 5 schematically shows an alternative embodiment of a device according to the present invention in side view.
Figs. 6A-C schematically show, in top view and in perspective, a counterweight as may be used in a device according to the present invention.
Fig. 7 schematically shows an imaging unit and associated units as may be used in a device of the present invention.

### Detailed description of embodiments

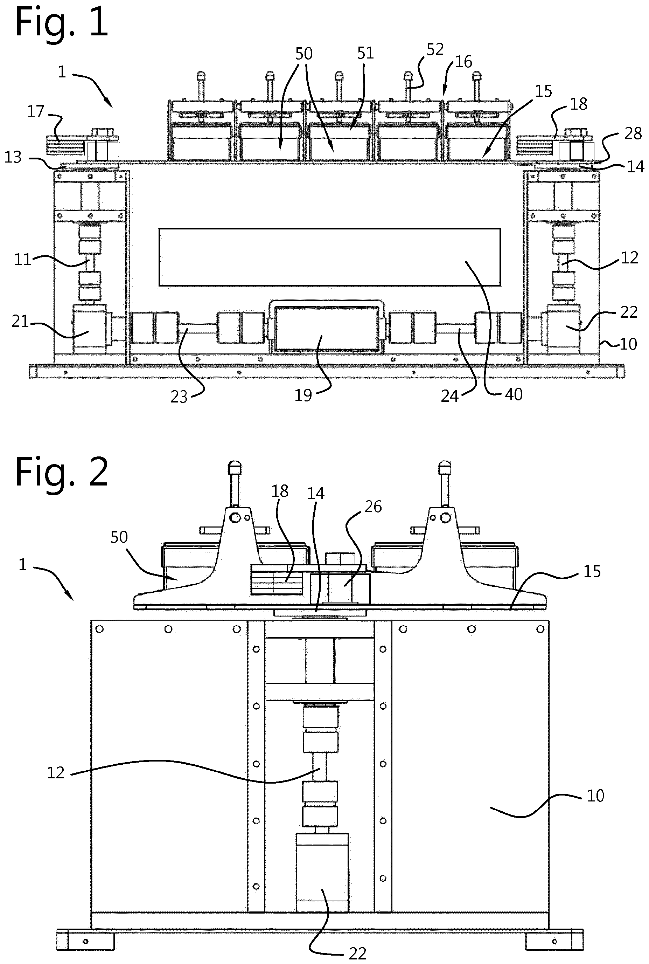

The merely exemplary embodiment of the device 1 shown in Fig. 1 comprises a frame 10 designed to accommodate and support the components of the device. The device 1 also comprises a platform 15 which is rotatably mounted on eccentric couplings 13 and 14 so as to be able to carry out an orbital movement relative to the frame 10.

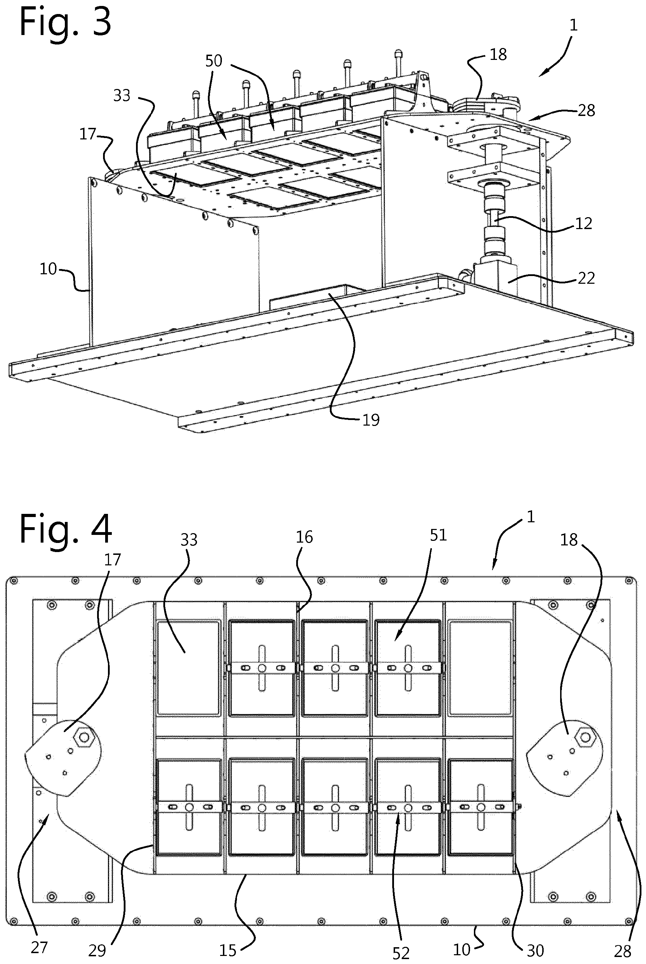

The platform 15 is arranged for receiving and accommodating containers 50, as will later be explained in more detail with reference to Figs. 3 & 4. In the embodiment shown, the containers 50 are provided with lids 51 which can be pressed against the containers 50 by positioning elements 52. Dividers 16, which extend perpendicularly (in operation: vertically) from the platform 15, provide support for the positioning elements 52 while creating compartments for receiving the containers.

A motor 19 drives the eccentric couplings 13 & 14 via horizontal axes 23 & 24, orthogonal gearboxes 21 & 22, and vertical axes 11 & 12. The eccentric couplings 13 & 14 transform the rotation of the axes 11 & 12 into an orbital movement of the platform 15. The orthogonal gearboxes 21 & 22, which may be known per se, transform the horizontal rotation of the axes 23 & 24 into a vertical rotation of the axes 11 & 12.

The arrangement of the motor 19 and the horizontal and vertical axes as shown in Fig. 1 offers the important advantage that the motor can be spaced apart from the platform so as to leave room for an imaging unit 40 underneath the platform. By adapting the frame 10 and the vertical axes 11 & 12, the size of the space available underneath the platform can be determined. Another important advantage of the arrangement of Fig. 1 is that the vertical axes 11 & 12 are located near the sides of the frame 10, away from the containers receiving area of the platform 15. The area below the containers receiving area of the platform 15 is therefore free from any parts, thus leaving room for the imaging unit 40.

In order to balance the orbital movement of the platform 15 and to counter vibrations of the device 1, counterweight units 17 & 18 are provided. In the embodiment of Fig. 1, the counterweights are mounted on the eccentric couplings 13 & 14 and are attached to these eccentric couplings by means of shafts or bearings (26 in Fig. 2) which allow a rotation of the platform while preventing a rotation of the counterweight units relative to the eccentric couplings.

It is noted that the amplitude of the platform 15 is defined by the spacing between the rotational axis of the eccentric coupling and the rotational axis of the shafts (26 in Fig. 2). The device of the present invention is preferably designed for an amplitude which is in a range from 3 to 50 mm, for example 20 mm or 30 mm. It will be understood that the amplitude can be adjusted by replacing the eccentric couplings.

By mounting the counterweight units on the eccentric couplings, either directly or indirectly via a shaft, two important advantages are achieved. A first advantage is that these counterweights can be positioned approximately at the level of the center of gravity of the loaded platform, as will be explained later in more detail. A second advantage is that the counterweights are spaced apart from the containers receiving part of the platform 15. More in particular, the counterweights are located to the sides of the platform 15 and do not obstruct the space above or below the platform 15, as in some prior art devices. In Fig. 1 the eccentric coupling 18 is shown to be located near an outer edge 28 of the platform 15, this will be explained in more detail later with reference to Fig. 4.

It can thus be seen that the present invention provides space underneath the movable platform by two related measures:
1. positioning the eccentric couplings, and hence the counterweights and any vertical axes away from the load bearing surface of the platform, and
2. positioning the counterweights approximately in the plane of the platform.
In addition, the embodiment of Fig. 1 advantageously utilizes the further measure of positioning the drive mechanism, such as the motor and the vertical axes, away from the platform. The space thus provided underneath the platform may advantageously be used to accommodate an imaging apparatus, such as a camera, for monitoring purposes, or an illumination apparatus, such as a set of lamps, for the cultivation of algae or plant cells.

It is noted that in the embodiment of Fig. 1 a single motor is used which drives the eccentric couplings via a series of axes. Alternative embodiments can be envisaged in which each eccentric coupling is directly driven by a respective motor, thus removing the need for the axes and the orthogonal gearboxes.

In embodiments having multiple motors the movement of the eccentric couplings is not mechanically synchronized, as in the embodiment of Fig. 1. Therefore, in such embodiments it is preferred to provide speed sensors (for example encoders) for sensing the rotational speed of the motors and a control unit for synchronizing the rotational speed of the motors.

The side view of Fig. 2 also shows the vertical axis 12 and the orthogonal gearbox 22 which drive the eccentric coupling 14. It can be seen that the eccentric coupling 14 comprises an axis 26 which is rotatably coupled with the platform 15 and which is offset relative to the vertical axis 12, thus causing an orbital movement of the platform 15. A counterweight unit 18 is shown to be mounted on the coupling 14.

The perspective view of Fig. 3 shows in particular the openings 33 in the platform 15 which allow the bottom side of the containers 50 to be imaged. For the sake of clarity, the imaging apparatus is not shown in Fig. 3. The openings 33 are slightly smaller than the containers 50, thus leaving a rim on all sides of an opening to support the container. Instead of an opening, a transparent part of the platform 15 may be provided to allow imaging.

A top view of the device 1 is shown in Fig. 4, where the openings 33 are also visible. In addition, Fig. 4 clearly shows the eccentric position of the counterweight units 17 & 18 which serves to compensate the eccentric movement of the platform 15.

Fig. 4 also shows the dividers 16 which extend from the platform 15 and which define compartments into which container units can be placed. Two such dividers define the limits 29 & 30 of the load bearing (that is, containing receiving) part of the platform 15. It can be seen that the counterweights 17 & 18, and hence the eccentric couplings, are positioned in the extensions of the platform 15, that is, in the parts of the platform beyond the limits 29 & 30 of the load bearing part. In the embodiment of Fig. 4, the eccentric couplings are located near the opposite outer edges 27 & 28 of the platform. It will be understood that the wording "near the outer edges" includes, but is not limited to, "within the outer 20%" or "within the outer 10%" of the platform. In general, it will be understood to mean "in the vicinity of the outer edges", and in particular "away from the load bearing part of the platform".

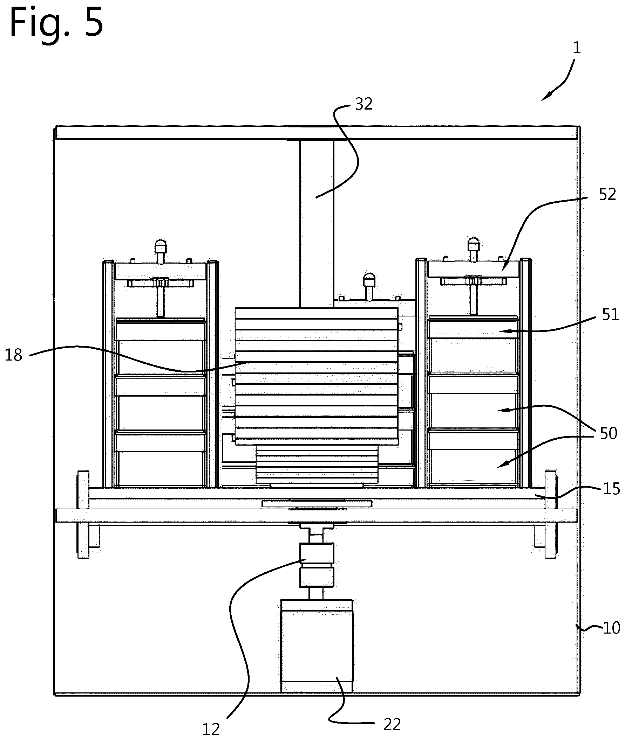

An alternative embodiment of the device according to the present invention is schematically illustrated in Fig. 5 in side view. Whereas the embodiment of Figs. 1-4 was designed for a single layer of containers, the embodiment of Fig. 5 is designed for multiple layers of containers, that is, for stacking containers. To this end, the positioning elements 52 are shown to be extended so as to be capable of positioning stacks of two, three, four or even more containers and preferably also for pressing the lids 51 on the containers 50. In addition, an axis 32 is provided as an extension of the axis 12 to provide additional support for the platform, which will carry a heavier load in this embodiment. The bottom end of the additional axis 32 will be mounted on the eccentric coupling 14 in such a way that the axis 32 will rotate normally, not eccentrically, while its top end will be rotatably fixed to the top part of the frame 10.

Another adaptation of this embodiment is the altered counterweight unit 18 (as well as 17, which is not shown, and any other counterweight unit the device may have). The more containers 50 are stacked on the platform 15, the higher the center of gravity of the moving components (containers plus platform) will be. An optimal compensation of the imbalance caused by the eccentric movement of the platform is however achieved when the counterweights are located at about the same height as the center of gravity of the moving components (containers plus platform). To be able to provide an optimal compensation with various loads, this embodiment has adjustable counterweight units. That is, the mass and the height of the counterweight unit can be adjusted to match the load.

In the embodiment of Fig. 5, the adjustable counterweight unit 18 is shown to consist of a stack of counterweights. This stack can be increased or decreased as desired by adding or removing counterweights. As more counterweights are added, the stack of counterweights will become higher and the center of gravity of the counterweight unit will also become higher. In this way, the added weight and increased height of stacks of containers can be matched and compensated. Preferably, the center of gravity of the counterweight units is approximately in the plane of the combined center of gravity of the loaded platform, that is, of the platform and the containers present on the platform. A step of placing one or more containers on the platform may therefore be followed by a step of adjusting the counterweight units by, for example, adding counterweights. Conversely, a step of removing one or more containers from the platform may be followed by a step of adjusting the counterweight units by, for example, removing counterweights.

In a preferred embodiment, the dimensions and the mass of each counterweight is chosen so as to match the dimensions and the mass of the average container, or of the average platform provided with containers. The counterweights are provided with suitable means for holding them in place when stacked.

In the embodiments shown, the shaker device has only a single platform, on which containers may or may not be stacked. Embodiments can be envisaged in which multiple platforms are used, arranged one above the other, and separated by pairs of shafts and eccentric couplings. That is, each platform may be carried by a pair of eccentric couplings from which shafts protrude which support the next level of eccentric couplings. By suitably arranging the eccentric couplings relative to each other, the successive platforms may make opposite rotational movements so as to counterbalance each other. In such embodiments, separate counterweight units would not be required.

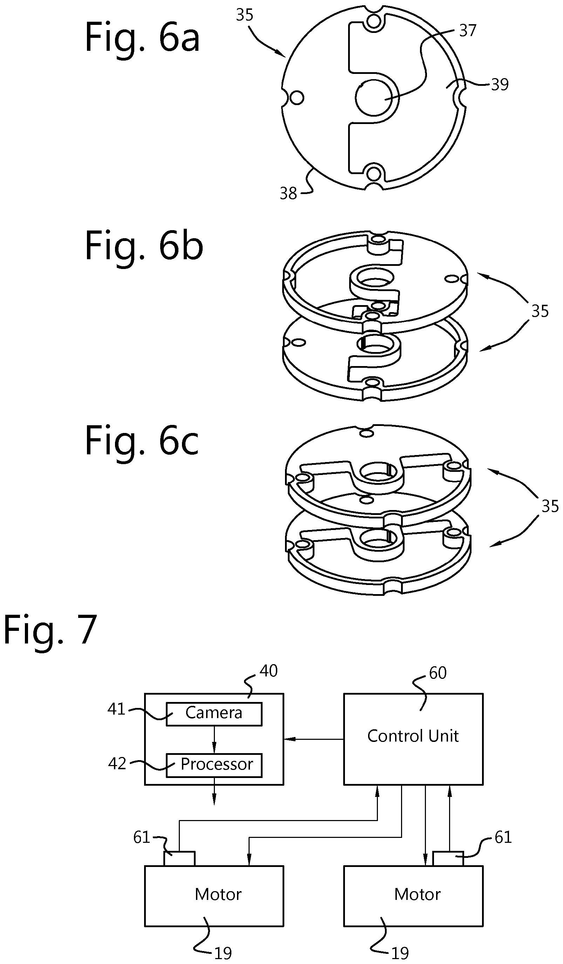

A top view of an eccentric coupling 14 with an adjustable counterweight unit 18 as may be used in the embodiment of Fig. 5 is presented in Figs. 6A-C.

The counterweight unit 18 of Fig. 5 is adjusted by adding or removing counterweights. In a particularly advantageous embodiment, at least some of the counterweights are not added or removed but may be rotated from a first position to a second position and vice versa. A suitable counterweight 35 is illustrated in Figs. 6A-C. This counterweight has an asymmetric mass: one part 38 is solid while another part 39 is empty. The counterweight 35 can be rotated about a central opening 37 suitable for receiving an axis (for example the shaft 32 shown in Fig. 5). In the first position, illustrated in Fig. 6B, the mass 38 of the two counterweight elements are located opposite each other, thus effectively canceling each other out. In the second position, illustrated in Fig. 6C, the mass 38 of the two counterweight elements are above each other, thus reinforcing each other. The counterweights may be provided with through holes, as illustrated in Figs. 6A-C, for receiving holding pins for preventing rotation and holding the counterweights in a selected position.

In an embodiment of the kind illustrated in Fig. 5, where multiple containers (e.g. microplates) 50 with their lids 51 are stacked, in particular if no imaging devices need to be present under the platform 15, it may be advantageous to drive the shafts 32 not from below, but from above, with one or two motors positioned above the containers, for example using two right-angled gear boxes that would then each be coupled to the top end of the shafts 32. In this manner, the shaker platform 15 is at a lower level, and so the total height of the orbital shaker device can be reduced.

In a particular advantageous embodiment, acceleration sensors are mounted on the frame 10 of the device for sensing any vibrations. A monitoring unit may be arranged for using the acceleration data produced by the sensors to indicate whether the counterweight units need adjusting.

Fig. 7 schematically shows a control unit 60, an imaging unit 40, and motors 19. The two electric motors 19 are each provided with a sensor 61 for sensing the rotational velocity of the respective motor. The sensor signals are sent to the control unit 60, which is arranged for comparing the sensor signals and ensuring the synchronization of the two motors. In other words, the control unit causes the motors to run at the same speed, at any speed. The control unit 60 may also be arranged for activating the imaging unit 40. In an advantageous embodiment, the control unit 60 reduces the speed of the motors prior to activating the imaging unit so as to provide a better image. If the imaging unit comprises a camera, such as the camera 41 illustrated in Fig. 7, then there is no need for the platform to come to a complete standstill and a reduced speed may be sufficient. Depending on the original orbital speed of the platform, a speed reduction may not be necessary. If the imaging unit 40 comprises a scanner, then a complete standstill may be required, as disclosed in more detail in International Patent Application WO 2010/037862. It will be understood that after imaging, the orbital speed may be brought back to its original value.

In the embodiment of Fig. 7, the imaging unit is provided with an image processor for processing and/or analyzing the images produced. Instead of, or in addition to, the imaging unit 40 an illumination unit (not shown) may be provided. An illumination unit may be arranged for providing a uniform illumination of the underside of the platform and may, for example, use LEDs.

The device of the present invention is capable of operating at shaking frequencies (that is, rotational velocities) in the biotechnological and biomedical range, that is, between approximately 150 and 1200 rpm (revolutions per minute), without undue vibrations. A preferred range is 200 to 700 rpm, more in particular 250 to 500 rpm, although other frequency ranges may also be used, depending on the particular application. It is noted that at an orbital movement amplitude of 50 mm a rotational speed of 200 to 300 rpm is generally preferred, while at an amplitude of 3 mm a speed of 800 to 1200 rpm is generally preferred.

The containers 50 are preferably microwell containers or micro titer plates configured for biotechnological and/or biomedical purposes, such as growing and/or sustaining bacteria and/or eukaryotic cell cultures. However, the device of the present invention may also be utilized in other fields of technology and the containers may be designed for containing paint, for example.

## Claims

1. An orbital shaker device (1) for biotechnological and/or biomedical applications, comprising:
- a frame (10),
- a platform (15) for receiving biotechnological and/or biomedical containers (50),
- eccentric couplings (13, 14) for allowing an orbital movement of the platform (15) relative to the frame (10),
- counterweight units (17, 18) for balancing the orbital movement, and
- at least one motor (19) for driving the eccentric couplings (13, 14),
- the device comprises two eccentric couplings (13, 14) arranged near respective opposite edges (27, 28) of the platform (15), **characterized in that**
- each counterweight unit (17, 18) is arranged approximately in the plane of the platform (15), and **in that**
- both eccentric couplings (13, 14) are driven by the at least one motor (19).

2. The device according to claim 1, wherein each eccentric coupling (13, 14) is driven via a respective vertical axis (11, 12), both axes being driven by a single motor (19).

3. The device according to claim 2, wherein the single motor (19) is connected to the axes (11, 12) through right-angle gear boxes (21, 22) or through a belt.

4. The device according to claim 1, wherein each eccentric coupling (13, 14) is driven by a separate motor (19), the device preferably comprising sensors (61) for sensing the speed of each motor.

5. The device according to any of the preceding claims, further comprising an imaging unit (40) located underneath the platform (15), said platform being provided with openings (33) and/or transparent parts allowing imaging the containers (50), wherein the imaging unit (40) preferably comprises a camera (41) and an image processor (42) for image processing and analysis.

6. The device according to claim 5, further comprising a control unit (60) for controlling the speed of the at least one motor (19), wherein the control unit (21) is arranged for reducing the speed of the at least one motor (19) in preparation for imaging.

7. The device according to any of the preceding claims, further comprising an illumination unit (40) located underneath the platform (15), said platform being provided with openings (33) and/or transparent parts allowing illuminating the containers (50).

8. The device according to any of the preceding claims, arranged for an orbital movement having an amplitude between 3 and 50 mm and a rotational speed between 150 and 1200 rpm.

9. The device according to any of the preceding claims, wherein the counterweight units (17, 18) are mounted on the eccentric couplings (13, 14), preferably via an axis (25, 26) to which the platform (15) is rotatably coupled.

10. The device according to any of the preceding claims, wherein the counterweight units (17, 18) are adjustable.

11. The device according to claim 10, wherein the counterweight units (17, 18) are arranged for adding and/or removing counterweights.

12. The device according to claim 10 or 11, wherein the counterweight units (17, 18) comprise counterweights (35) having an asymmetrical mass distribution relative to an axis (37) and which can be rotated about said axis (37).

13. The device according to any of the preceding claims, further comprising one or more containers (50) arranged on the platform (15), wherein the center of gravity of each counterweight unit (17, 18) is arranged approximately in the plane of the combined center of gravity of the platform (15) and the containers (50).

14. A use of an orbital shaker device (1) according to any of claims 10 to 12, comprising the steps of:
- placing at least one container (50) on the platform (15) or removing at least one container from the platform, and
- adjusting the counterweight units (17, 18).

15. The use according to claim 14, comprising the step of adding or removing counterweights (35).

## Patentansprüche

1. Eine Orbitalschüttlervorrichtung (1) für biotechnologische und/oder biomedizinische Anwendungen, umfassend:
- einen Rahmen (10),
- eine Plattform (15) zum Aufnehmen biotechnologischer und/oder biomedizinischer Behälter (50),
- exzentrische Kupplungen (13, 14) zum Zulassen einer orbitalen Bewegung der Plattform (15) relativ zum Rahmen (10),
- Gegengewichtseinheiten (17, 18) zum Ausgleichen der orbitalen Bewegung und
- zumindest einen Motor (19) zum Antreiben der exzentrischen Kupplungen (13, 14),
- wobei die Vorrichtung zwei exzentrische Kupplungen (13, 14) umfasst, die nahe entsprechenden gegenüberliegenden Rändern (27, 28) der Plattform (15) angeordnet sind, **dadurch gekennzeichnet, dass**
- jede Gegengewichtseinheit (17, 18) ungefähr in der Ebene der Plattform (15) angeordnet ist, und dass
- beide exzentrischen Kupplungen (13, 14) durch den zumindest einen Motor (19) angetrieben sind.

2. Die Vorrichtung nach Anspruch 1, wobei jede exzentrische Kupplung (13, 14) über eine entsprechende vertikale Achse (11, 12) angetrieben wird, wobei beide Achsen durch einen einzigen Motor (19) angetrieben werden.

3. Die Vorrichtung nach Anspruch 2, wobei der einzige Motor (19) mit den Achsen (11, 12) durch rechtwinkelige Getriebekästen (21, 22) oder durch einen Riemen verbunden ist.

4. Die Vorrichtung nach Anspruch 1, wobei jede exzentrische Kupplung (13, 14) durch einen separaten Motor (19) angetrieben wird, wobei die Vorrichtung vorzugsweise Sensoren (61) zum Erfassen der Geschwindigkeit jedes Motors umfasst.

5. Die Vorrichtung nach einem der vorstehenden Ansprüche, weiter umfassend eine Bildgebungseinheit (40), die unterhalb der Plattform (15) gelegen ist, wobei die Plattform mit Öffnungen (33) und/oder transparenten Teilen versehen ist, die eine Abbildung der Behälter (50) erlauben, wobei die Bildgebungseinheit (40) vorzugsweise eine Kamera (41) und einen Bildprozessor (42) zur Bildverarbeitung und Analyse umfasst.

6. Die Vorrichtung nach Anspruch 5, weiter umfassend eine Steuereinheit (60) zum Steuern der Geschwindigkeit des zumindest einen Motors (19), wobei die Steuereinheit (21) zum Verringern der Geschwindigkeit des zumindest einen Motors (19) bei der Vorbereitung zur Bildgebung angeordnet ist.

7. Die Vorrichtung nach einem der vorstehenden Ansprüche, weiter umfassend eine Beleuchtungseinheit (40), die unterhalb der Plattform (15) gelegen ist, wobei die Plattform mit Öffnungen (33) und/oder transparenten Teilen versehen ist, die eine Beleuchtung der Behälter (50) erlauben.

8. Die Vorrichtung nach einem der vorstehenden Ansprüche, die für eine orbitale Bewegung mit einer Amplitude zwischen 3 und 50 mm und einer Drehgeschwindigkeit zwischen 150 und 1200 U/min angeordnet ist.

9. Die Vorrichtung nach einem der vorstehenden Ansprüche, wobei die Gegengewichtseinheiten (17, 18) an den exzentrischen Kupplungen (13, 14) vorzugsweise über eine Achse (25, 26) montiert sind, an die die Plattform (15) drehbar gekoppelt ist.

10. Die Vorrichtung nach einem der vorstehenden Ansprüche, wobei die Gegengewichtseinheiten (17, 18) einstellbar sind.

11. Die Vorrichtung nach Anspruch 10, wobei die Gegengewichtseinheiten (17, 18) zum Hinzufügen und/oder Entfernen von Gegengewichten angeordnet sind.

12. Die Vorrichtung nach Anspruch 10 oder 11, wobei die Gegengewichtseinheiten (17, 18) Gegengewichte (35) mit einer asymmetrischen Masseverteilung relativ zu einer Achse (37) umfassen und die um die Achse (37) gedreht werden können.

13. Die Vorrichtung nach einem der vorstehenden Ansprüche, weiter umfassend einen oder mehrere Behälter (50), die auf der Plattform (15) angeordnet sind, wobei der Schwerpunkt jeder Gegengewichtseinheit (17, 18) ungefähr in der Ebene des kombinierten Schwerpunkts der Plattform (15) und der Behälter (50) angeordnet ist.

14. Eine Verwendung einer Orbitalschüttlervorrichtung (1) nach einem der Ansprüche 10 bis 12, umfassend die Schritte:
- Anordnen zumindest eines Behälters (50) auf der Plattform (15) oder Entfernen zumindest eines Behälters von der Plattform und
- Einstellen der Gegengewichtseinheiten (17, 18).

15. Die Verwendung nach Anspruch 14, umfassend den Schritt zum Hinzufügen oder Entfernen von Gegengewichten (35).

## Revendications

1. Dispositif secoueur orbital (1) pour des applications biotechnologiques et/biomédicales, comprenant :
- un châssis (10),
- une plateforme (15) pour recevoir des récipients biotechnologiques et/ou biomédicaux (50),
- des couplages excentriques (13, 14) pour permettre un mouvement orbital de la plateforme (15) par rapport au châssis (10),
- des unités de contrepoids (17, 18) pour équilibrer le mouvement orbital et
- au moins un moteur (19) pour entraîner les couplages excentriques (13, 14),
- le dispositif comprenant deux couplages excentriques (13, 14) agencés près de bords opposés respectifs (27, 28) de la plateforme (15), **caractérisé en ce que**
- chaque unité de contrepoids (17, 18) est agencée approximativement dans le plan de la plateforme (15) et
- les deux couplages excentriques (13, 14) sont entraînés par le au moins un moteur (19).

2. Dispositif selon la revendication 1, dans lequel chaque couplage excentrique (13, 14) est entraîné via un axe vertical respectif (11, 12), les deux axes étant entraînés par un seul moteur (19).

3. Dispositif selon la revendication 2, dans lequel le moteur unique (19) est raccordé aux axes (11, 12) via des boîtes d'engrenages à angle droit (21, 22) ou via une courroie.

4. Dispositif selon la revendication 1, dans lequel chaque couplage excentrique (13, 14) est entraîné par un moteur séparé (19), le dispositif comprenant de préférence des capteurs (61) pour capter la vitesse de chaque moteur.

5. Dispositif selon l'une quelconque des revendications précédentes, comprenant en outre une unité d'imagerie (40) située en dessous de la plateforme (15), ladite plateforme étant pourvue d'ouvertures (33) et/ou de parties transparentes permettant d'imager les récipients (50), dans lequel l'unité d'image (40) comprend de préférence une caméra (41) et un processeur d'image (42) pour le traitement et l'analyse de l'image.

6. Dispositif selon la revendication 5, comprenant en outre une unité de commande (60) pour commander la vitesse du au moins un moteur (19), dans lequel l'unité de commande (21) est agencée pour réduire la vitesse du au moins un moteur (19) en préparation de l'imagerie.

7. Dispositif selon l'une quelconque des revendications précédentes, comprenant en outre une unité d'éclairage (40) située en dessous de la plateforme (15), ladite plateforme étant pourvue d'ouvertures (33) et/ou de parties transparentes permettant l'éclairage des récipients (50).

8. Dispositif selon l'une quelconque des revendications précédentes, agencée pour un mouvement orbital ayant une amplitude entre 3 et 50 mm et une vitesse de rotation entre 150 et 1 200 tr/min.

9. Dispositif selon l'une quelconque des revendications précédentes, dans lequel les unités de contrepoids (17, 18) sont montées sur les couplages excentriques (13, 14), de préférence via un axe (25, 26) auquel la plateforme (15) est couplée à rotation.

10. Dispositif selon l'une quelconque des revendications précédentes, dans lequel les unités de contrepoids (17, 18) sont ajustables.

11. Dispositif selon la revendication 10, dans lequel les unités de contrepoids (17, 18) sont agencées pour ajouter et/ou retrancher des contrepoids.

12. Dispositif selon la revendication 10 ou 11, dans lequel les unités de contrepoids (17, 18) comprennent des contrepoids (35) ayant une distribution de masse asymétrique par rapport à un axe (37) et qui peuvent être soumis à une rotation autour dudit axe (37).

13. Dispositif selon l'une quelconque des revendications précédentes, comprenant en outre un ou plusieurs récipients (50) agencés sur la plateforme (15), dans lequel le centre de gravité de chaque unité de contrepoids (17, 18) est agencé approximativement dans le plan du centre de gravité combiné de la plateforme (15) et des récipients (50).

14. Utilisation d'un dispositif secoueur orbital (1) selon l'une quelconque des revendications 10 à 12, comprenant les étapes consistant à :
- placer au moins un récipient (50) sur la plateforme (15) ou retirer au moins un récipient de la plateforme et
- ajuster les unités de contrepoids (17, 18).

15. Utilisation selon la revendication 14, comprenant l'étape d'addition ou de retrait de contrepoids (35).
